Europäisches Patentamt

European Patent Office

Office européen des brevets

(11) Numéro de publication: **0 151 102**
A2

# (12) DEMANDE DE BREVET EUROPEEN

(21) Numéro de dépôt: 85870014.9

(22) Date de dépôt: 28.01.85

(51) Int. Cl.⁴: **C 12 N 15/00**, C 12 P 21/02

(30) Priorité: 30.01.84 US 575122

(71) Demandeur: **Smith Kline - RIT Société anonyme dite:,
rue du Tilleul, 13, B-1320 Genval (Rixensart) (BE)**

(43) Date de publication de la demande: **07.08.85**
*Bulletin 85/32*

(72) Inventeur: **Cabezon, Teresa, Rue de Terheyde, 78,
B-1640 Rhode st Genèse (BE)**
Inventeur: **Bollen, Alex Joseph, Gaasbeekstraat, 65,
B-1711 Itterbeek (BE)**

(84) Etats contractants désignés: **AT BE CH DE FR GB IT LI
LU NL SE**

(74) Mandataire: **Tasset, Gérard, SMITHKLINE - RIT rue de
l'Institut, 89, B-1330 Rixensart (BE)**

(54) **Expression de l'alpha-1-antitrypsine humaine dans la levure.**

(57) Le gène de structure pour l'α-1-antitrypsine humaine est fusionné en phase au promoteur du gène de l'Arg 3 et exprimé dans la levure.

EP 0 151 102 A2

## EXPRESSION DE L'ALPHA-1-ANTITRYPSINE HUMAINE
## DANS LA LEVURE

### Etendue de l'invention

La présente invention est relative à la préparation d'une molécule d'ADN recombinante comprenant une séquence nucléotidique codant pour l'alpha-1-antitrypsine humaine dans la levure.

### Information de base

L'alpha-1-antitrypsine humaine (AATh) est une alpha-1-globuline présente dans le sérum et divers autres liquides organiques. Synthétisée dans le foie, l'AATh est une glycoprotéine présentant un poids moléculaire d'environ 50000 à 55000 daltons. Les enzymes protéolytiques inhibés par l'AATh comprennent la trypsine, la chymotrypsine, l'élastase pancréatique, la collagénase cutanée, la rénine et l'urokinase ainsi que les protéases des lymphocytes polynucléaires. La déficience héréditaire en AATh chez les humains est associée à divers états pathologiques, en particulier, l'emphysème et la maladie du foie. Voir, par exemple, Morse, N. Eng. J. Med. 299 (19):1045-1048 (1978) et 299 (20):1099-1105 (1978); Tobin et al, Arch. Int. Med. 143 (7):1342-1348 (1982); et Carrell et al, Nature 298 (5872):329-334 (1982).

L'élastase est une protéinase qui dégrade le tissu pulmonaire. Incontrôlée, son activité peut engendrer l'emphysème. Gadek et al, Am. Rev. Respir. Dis. 127:S45-S46 (1983) et Glaser et al, Am. Rev. Respir. Dis. 127:547-553 (1983), par exemple, ont montré que l'AATh peut s'avérer thérapeutiquement utile dans le traitement de l'emphysème.

Vu son utilité thérapeutique, les chercheurs ont été amenés à la recherche de techniques en vue de produire l'AATh en grandes quantités. Traditionnellement, les techniques de ce genre ont comporté la purification de l'AATh à partir du plasma sanguin. Voir, par exemple, Coan et al, Brevet des Etats-Unis d'Amérique n° 4379087.

Dans Genetic Engineering News 2(6):29 (1982) et Drug Cosmet. Ind. 131(5):32-36 (1982), il est rapporté que l'AATh a été exprimée dans la levure, Saccharomyces cerevisiae.

Kawasaki, et al, "The Molecular Biology of Yeast", Août 16-21, 1983, Cold Spring Harbor Laboratory, mentionnent que l'AATh a été exprimée dans la levure à partir du promoteur du gène de la triose phosphate isomérase.

Bollen et al., brevet belge n° 895961 décrivent le clonage de l'AATh dans E. coli sur un plasmide, pULB1523, comprenant un fragment de ADNc de 1400 paires de bases (pb) transcrit inversément à partir du poly-AARN en provenance de cellules hépatiques humaines clonées dans le site PstI du pBR322. Bollen et al. révèlent que le fragment ADNc comprend tout le gène AATh, incluant son propre codon d'initiation de la traduction.

Long et al., Structure and Organization of Genes I, Abstract n° 25 (1983), mentionnent le clonage et le séquençage de l'AATh.

Divers vecteurs sont connus et disponibles en vue du clonage et/ou de l'expression des fragments d'ADN dans la levure et/ou dans E. coli. Broach et al, Gene 8:121-133 (1979), signalent le YEp13, un vecteur navette de E. coli-levure déposé à l'American Type Culture Collection (ATCC) sous le numéro d'ordre 37115. Crabeel et al, Proc. Natl. Acad. Sci. USA 78:5026-5030 (1981) signalent le plasmide pYeUra3Arg3. Boyer et al, Gene 2:75 (1977), signalent le plasmide pBR32 de E. coli 2 déposé à l'ATCC sous le numéro d'ordre 37017.

## Résumé de l'invention

Un premier aspect de l'invention concerne une molécule d'ADN recombinante comprenant une séquence nucléotidique qui code pour l'alpha-1-antitrypsine humaine (AATh) et une région de régulation qui peut effectuer la transcription de la séquence AATh dans la levure. Selon des modes de réalisation particuliers de l'invention, la région de régulation provient du gène Arg3 de la levure et/ou la séquence AATh code pour la pré-AATh ou l'AATh mature.

Un autre aspect de la présente invention concerne un microorganisme transformé à l'aide de la molécule d'ADN recombinante conforme à la présente invention.

Un autre aspect encore de la présente invention concerne un vecteur d'expression plasmidique pour levure, qui peut être utilisé, par exemple, pour exprimer l'AATh, comprenant les régions du promoteur et du terminateur de la transcription de l'Arg3 et ayant un site de restriction endonucléasique entre les régions du promoteur et du terminateur pour l'insertion et l'expression d'une séquence codante.

## Brève description des figures

La figure 1 représente un diagramme illustrant la construction du pRIT10749 qui porte le gène Arg3.

La figure 2 représente un diagramme illustrant la construction des plasmides intermédiaires pRIT10772 et pRIT10773.

La figure 3 représente un diagramme illustrant la construction du pRIT10782 qui porte les régions de régulation de l'Arg3 et une séquence nucléotidique de l'AATh.

La figure 4 représente un diagramme illustrant la construction d'un plasmide intermédiaire, le pRIT10779.

- 4 -                    0151102

## Description détaillée de l'invention

Dans la molécule d'ADN recombinante conforme à la présente invention, la séquence codant pour l'AATh est favorisée par un promoteur de levure. La demanderesse a découvert que la région du promoteur de l'Arg3 est utile à cette fin. Le promoteur de l'Arg3 peut provenir du pYeUra3Arg3, un plasmide décrit par Crabeel et al., Proc. Natl. Acad. Sci. USA 78:5026-5030 (1981). Le gène Arg3 est porté sur un fragment HindIII de celui-ci. Le gène de structure Arg3 code pour l'ornithine carbamoyl transférase (OCT). La région du promoteur peut être isolée, de façon commode, sur un fragment XhoI-HincII, le site HincII étant environ deux nucléotides en aval du Arg3ATG. Une région portant le terminateur de transcription de l'Arg3 peut être isolée, de façon commode, sur un fragment HaeIII-HindIII. Le terminateur de transcription (tt) est supposé stabiliser la transcription.

Un fragment d'ADN portant la séquence codant pour l'AATh peut provenir des ARN messagers totaux de foie humain (mARN) selon des techniques connues, telles que décrites par Bollen et al, dans le brevet belge n° 895961 auquel on se référera ici plus en détail. Un procédé caractéristique implique le passage de l'ARN total de foie sur une colonne d'oligo(dt)-cellulose afin de récupérer le polyA-ARN, c'est-à-dire, le mARN. Le mARN total est fractionné en fonction de la taille, les fractions contenant un ARN messager d'environ 14 à 15S étant recueillies sur des gradients de densité en saccharose. L'ADN complémentaire (ADNc) est préparé, in vitro, au départ d'une fraction de ce genre via une transcription inverse par traitement à l'aide d'une transcriptase réverse. Le simple brin de ADNc est transformé en double brin par traitement avec une polymérase. Les extrémités de la population hétérogène du ADNc sont rendues franches, par exemple, à l'aide de la S1 nucléase et elles sont fractionnées de façon à isoler des fractions de 1100 paires de bases (pb) ou plus. Les fractions isolées peuvent être clonées et exprimées de façon à confirmer l'obtention d'une séquence nucléotidique AATh. Bollen et al. ont allongé les extrémités 3' avec des nucléotides G de fractions isolées et ont cloné celles-ci dans le site PstI du pBR322. Le dérivé pBR322 portant la séquence AATh a été identifié comme étant pULB1523.

Ainsi qu'il a été mis en évidence par Leicht et al, Nature 297:655-659 (1982), les trois premiers acides aminés de l'AATh mature sont l'acide glutamique, l'acide aspartique et la proline. En conséquence, un site BamHI est localisé entre les codons pour l'acide glutamique et l'aspartique comme suit :

GAG'GAT CCC

Glu Asp Pro.

Donc, une séquence codant pour l'AATh mature peut être obtenue par restriction d'une séquence codant pour la pré-AATh, comme le fragment PstI du pULB1523, avec BamHI, en admettant évidemment que l'AATh mature ne présente pas d'acide glutamique N-terminal. Cette distinction est acceptable parce que l'AATh mature ne présentant pas d'acide glutamique N-terminal est censé avoir la même activité que l'AATh mature naturelle.

Une molécule d'ADN recombinante comprenant la région du promoteur de l'Arg3 et le gène AATh peut être un vecteur, par exemple, un plasmide. De tels vecteurs peuvent être construits par ligation d'une fusion comprenant le promoteur de l'Arg3 et les séquences AATh avec une région de réplication, comme, par exemple, l'origine plasmidique de réplication du plasmide 2 u. De tels vecteurs peuvent être utilisés pour produire l'AATh destinée au traitement de l'emphysème et d'autres états en rapport avec la carence en AATh. Les régions de l'Arg3 contenues dans ces vecteurs peuvent être utilisées dans d'autres vecteurs par sous-clonage ou par reconstruction des régions ou, en substance, des mêmes régions, c'est-à-dire, des régions qui peuvent différer par l'addition, la suppression ou la substitution d'une ou quelques bases mais qui ne sont pas essentiellement modifiées en fonction de leur activité. Des modes de réalisation particuliers de plasmides exemplifiés conformes à la présente invention et qui portent des séquences codant pour l'AATh sont le pRIT10782 et le pRIT10787.

Les vecteurs d'expression comprenant les régions du promoteur et du terminateur de la transcription de l'Arg3 peuvent être préparés selon des procédés connus à l'aide de matériaux disponibles bien connus. Des modes de réalisation particuliers exemplifiés de vecteurs d'expression plasmidiques conformes à la présente invention sont le pRIT10772, le pRIT10774 et le pRIT 10779. Ces plasmides et d'autres caractéristiques de l'invention sont décrits plus en détails dans les exemples illustratifs non limitatifs suivants. Dans tous les exemples, la transformation de la levure a été effectuée, en substance, comme il est décrit par Hinnen et al., Proc. Natl. Acad. Sci. USA 75:1929-1933 (1978) et la transformation de E. coli a été effectuée, en substance, comme il est décrit dans "Molecular cloning", Maniatis et al., Cold Spring Harbour Laboratory, New York (1982), pp. 249-251. Tous les exemples ont été réalisés à l'aide d'enzymes et de réactifs disponibles dans le commerce, en substance, conformément aux instructions des vendeurs et à l'aide de produits de départ disponibles bien connus.

EXEMPLES

Exemple 1

Plasmide pRIT10774 intermédiaire

Le pRIT10774 est un vecteur navette levure-E. coli portant une région promoteur et une région terminateur de la transcription (tt) au départ du gène Arg3 avec un site BamHI dans l'intervalle. Le plasmide n'a pas un codon d'initiation de la transcription (ATG). Il a été préparé comme suit.

A. pMC200

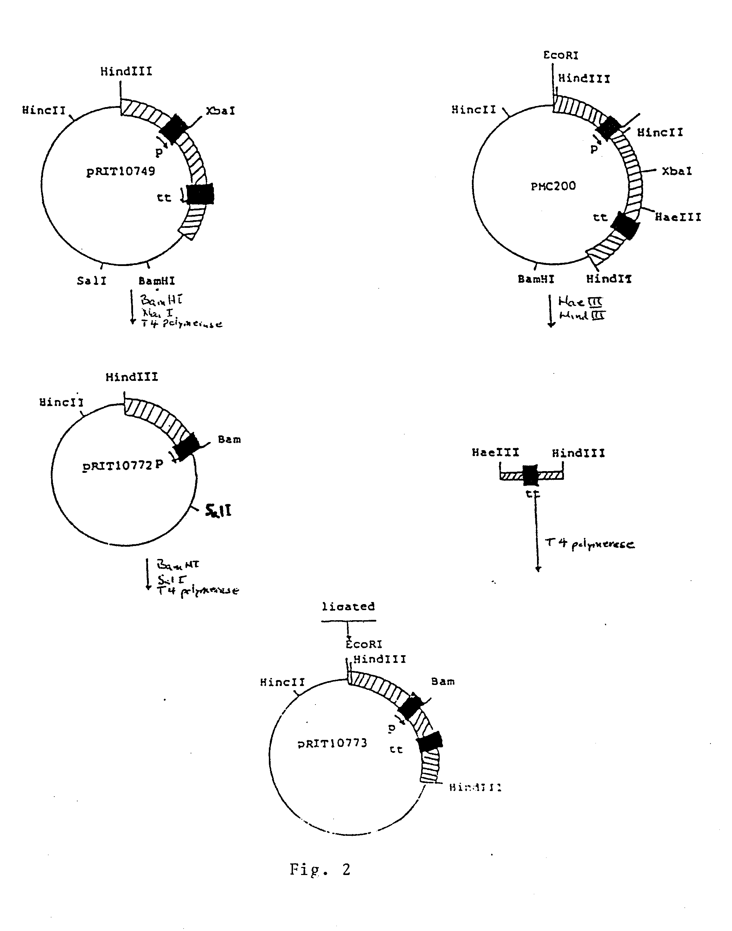

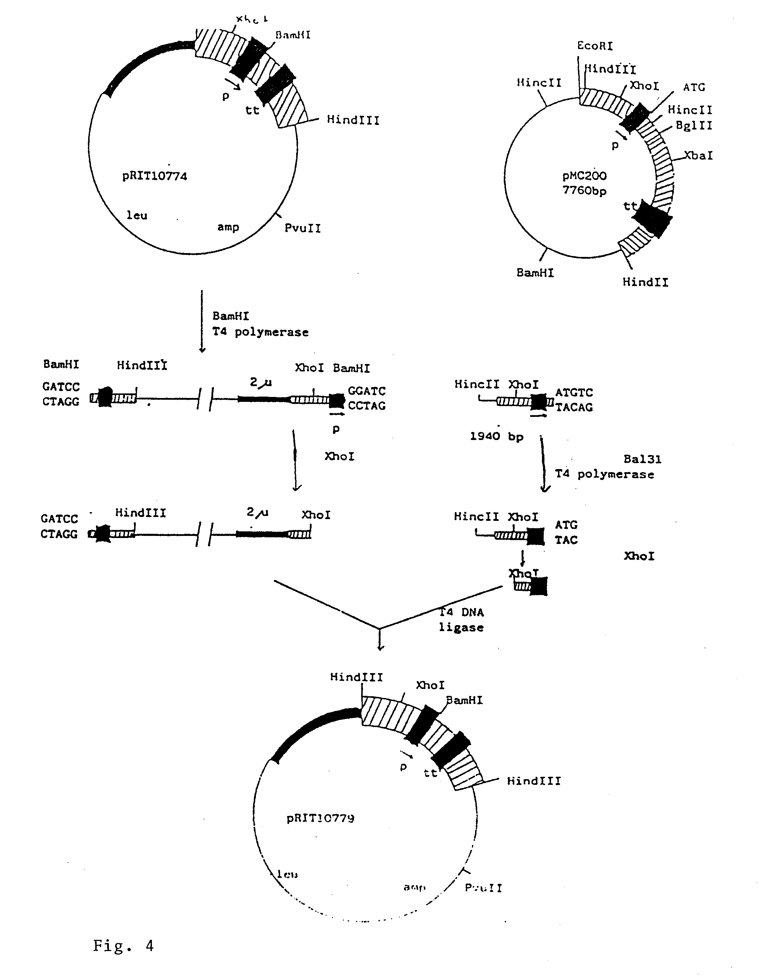

Un fragment d'ADN de levure de 3300pb désignant le gène de l'Arg3 a été obtenu par digestion du pYeUrea3Arg3 avec HindIII. Le plasmide pYeUrea3Arg3 a été décrit par Crabeel et al., Proc. Natl. Acad. Sci. USA 78:5026 (1981). Le fragment de 3300 pb a été cloné dans le site HindII du pBR322 et transformé dans la souche MM294 de E. coli K12. Les transformants ont été sélectionnés sur milieu d'ampicilline et criblés en fonction de leur résistance à la tétracycline. On a constaté qu'une colonie transformée contenait un plasmide, le pMC200, qui se compose du pBR322 avec un insert au site Hind III. Deux paires de bases en aval (3') de ATG de l'Arg3 constituent un site HincII. Voir figures 2 et 4. Le plasmide pMC200 a été déposé conformément aux dispositions du traité de Budapest à l'American Type Culture Collection, Rockville, Maryland, E.U.A. sous le numéro d'ordre 39130.

B. pRIT10749

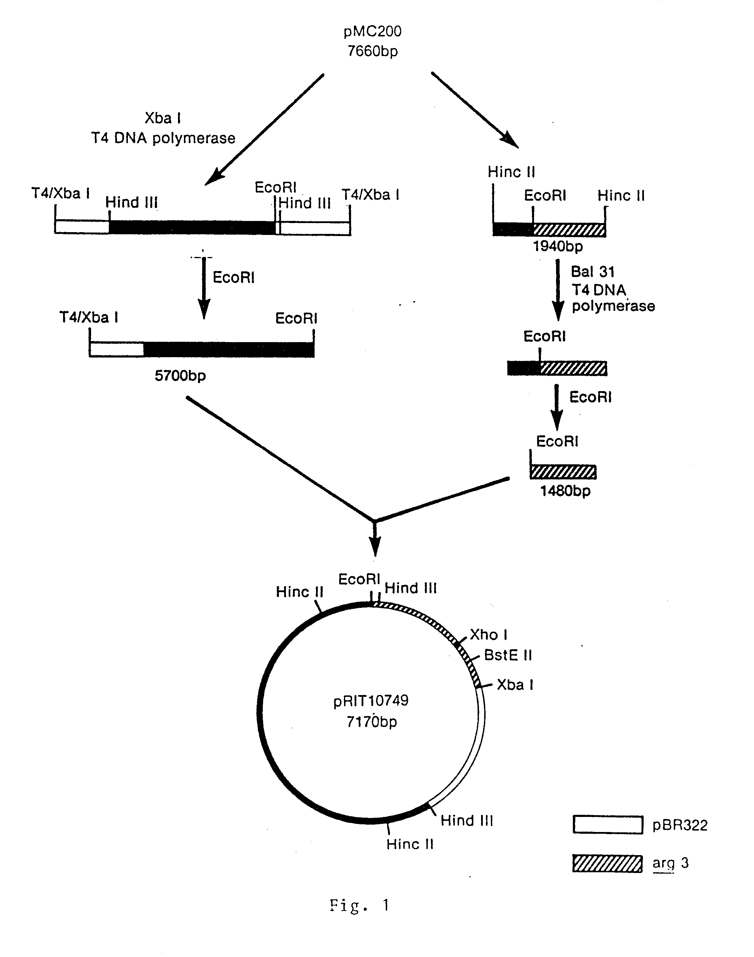

(Voir fig. 1) Le pMC200 purifié a été digéré avec HincII et soumis à l'électrophorèse sur un gel d'acrylamide à 10 %. Un fragment de 1940 pb contenant la région de régulation de l'Arg3 a été récupéré du gel par électroélution et précipitation à l'éthanol. Environ 4 μg de fragment a été incubé avec 0,2 unité d'exonucléase Bal31 pendant 1 à 3 secondes à 30° C dans

100 μl de tampon (pH 8) contenant 12,5 mM de MgCl$_2$, 12,5 mM de CaCl$_2$, 200 mM de NaCl, 1 mM d'éthylènedi-amine tétraacétate (EDTA) et 200 mM de trométhamine-HCl. L'ADN traité a été extrait au phénol et précipité avec de l'éthanol. Une aliquote de 1 μg a été incubée avec l'ADN polymérase de T4 en présence de déoxynucléotide triphosphates afin de réparer toutes extrémités simple brin et a été ensuite digéré avec l'endonucléase EcoRI. Ce procédé a produit des fragments d'ADN de longueurs variables en raison de la résection avec Bal31. Chaque fragment avait une extension EcoRI fixe à une extrémité, l'autre extrémité étant à extrémité à bouts francs et située à quelque distance du site d'origine HincII et de l'ATG de l'Arg3. Des fragments d'environ 1480 pb contenant la région de régulation de l'Arg3 ont été isolés par électrophorèse sur un gel d'acrylamide à 7,5 % suivie d'électroélution et d'une précipitation à l'éthanol. Cette région de régulation réduite est préférée parce qu'elle peut promouvoir la transcription conduisant à la synthèse d'une protéine dépourvue de séquences en acides aminés OCT.

Dans une seconde série de réactions, le pMC200 a été digéré avec XbaI et les extrémités simple brin en 5' ont été complétées ou transformées en extrémité à bouts francs par incubation avec l'ADN polymérase de T4 et les déoxynucléotide trisphosphates. Cet ADN a été digéré avec EcoRI et un grand fragment EcoRI-T4/XbaI d'environ 5700 pb contenant des séquences d'ADN de levure situées en 3' par rapport au site XbaI, plus des séquences pBR322 a été purifié par électrophorèse, électroélution et précipitation à l'éthanol. Ce fragment a été mélangé et ligué aux fragments de 1480 pb contenant le promoteur.

Le mélange de ligation a été utilisé pour transformer les cellules compétentes de la souche MM294 de

0151102

E. coli K12. Les transformants ont été sélectionnés sur gélose d'ampicilline. L'ADN plasmidique a été isolé des colonies de transformants, en substance, selon le procédé décrit par Birnboim et al., Nucl. Acid. Res. 7:1513 (1979) et ceux-ci ont été criblés pour la présence d'un site XbaI par digestion avec XbaI. Un site XbaI était présent uniquement dans les plasmides dans lesquels l'extrémité XbaI réparée, en provenance de la seconde série de réactions, a été liguée avec un autre fragment provenant de la première série qui porte un résidu T à l'extrémité 3' de façon à rétablir une séquence reconnue par l'enzyme XbaI, TCTAGA, dans une nouvelle position. Un plasmide possédant le site XbaI placé à neuf nucléotides en amont du site HincII original dans l'insert ADN de levure pMC200 a été identifié comme étant le pRIT10749. En d'autres mots, le promoteur est porté sur un fragment HindIII-HincII provenant du pYeUra3Arg3, ayant environ 13 nucléotides supprimés en 5' au site HincII afin de se terminer en un résidu T. Les nucléotides supprimés comprennent l'ATG de l'Arg3. Le résidu T est fusionné au site XbaI réparé en aval duquel se trouvent les séquences OCT additionnelles et le terminateur de transcription.

Un échantillon du pRIT10749 a été digéré avec une combinaison de BstEII et de XbaI. La taille de la région de régulation de l'Arg3 supprimée a été évaluée à environ 210 pb par comparaison avec le pMC200 qui a été digéré de façon similaire avec BstEII et XbaI et par référence aux poids moléculaires connus des fragments d'ADN du phage $\Phi$ iX174 décrit par Fuchs et al., Gene 4:1 (1978). Le plasmide pRIT 10749 a été déposé conformément aux dispositions du Traité de Budapest à l'American Type Culture Collection, Rockville, Maryland, E.U.A. sous le numéro d'accès 39133.

## C. pRIT10772

(Voir fig. 2) Le plasmide pRIT10749 a été digéré avec les endonucléases de restriction BamHI et XbaI. Le plus grand fragment résultant (5560 pb) a été purifié par électrophorèse dans 1 % d'agarose suivie par électroélution de l'ADN en provenance du gel. Une aliquote de 1 µg a été traitée par l'ADN polymérase de T4 en présence de déoxyribonucléotide triphosphate de façon à compléter l'extension à simple brin et a été circularisée par incubation pendant 16 h à 16° C en présence d'ADN ligase de T4. Cette préparation a été utilisée pour transformer la souche E. coli MM294 et la rendre résistante à l'ampicilline. Un des transformants qui contenait un plasmide muni d'un site BamHI adjacent à la région du promoteur de l'Arg3 a été sélectionné. Ce plasmide, pRIT10772XbaI-BamHI, a été appelé pRIT10772.

## D. pRIT 10773

(Voir fig. 2) Le plasmide pRIT10772 a été digéré avec BamHI et SalI et à nouveau, le plus grand fragment (5250 pb) a été purifié par électrophorèse suivie d'une électroélution. Parallèlement, le plasmide pMC200 a été digéré avec HaeIII et HindIII et un fragment de 1300 pb contenant la région de terminaison de la transcription du gène Arg3 a été purifié par électrophorèse sur un gel d'acrylamide à 10 % suivie d'une électroélution.

Cent ng de fragment BamHI-SalI et 100 ng de fragment HaeIII-HindIII du pMC200 ont été mélangés, incubés avec de l'ADN polymérase de T4 de façon à remplir les extensions 5' et ligués pendant 16 h à 16° C avec de l'ADN ligase de T4. Le mélange a été utilisé pour transformer la souche E. coli MM294 et la rendre résistante à l'ampicilline. Les plasmides de plusieurs transformants ont été analysés et on a conservé l'un d'eux, le pRIT10773, dans lequel l'insertion du fragment HaeIII-HindIII se trouvait dans l'orientation correcte. Le plasmide pRIT10773 conserve un site BamHI entre les régions du promoteur et de la terminaison de la transcription de l'Arg3, c'est-à-dire, au lieu de fusion des sites BamHI et HaeIII, ainsi qu'un site HindIII à la jonction HindIII-SalI. Dans ce plasmide, la majeure partie de la région codant pour l'Arg3 inutile pour l'expression des gènes étrangers a été éliminée. La séquence à proximité du site BamHI est CCCATCAACTTGTACACTCGTCTAG'GATCC.

## E. YEp13 Δ HindIII

Le vecteur YEp13 est un vecteur navette E. coli-S. cerevisiae. Il a été décrit par Broach et al, Gene 8:121 (1979) et est disponible à l'ATCC sous le numéro d'ordre 37115. Un petit fragment HindIII a été excisé du plasmide par digestion avec HindIII et le plasmide a été religué pour préparer un plasmide dérivé, le YEp13 Δ HindIII qui contient un site HindIII unique.

## F. pRIT10788

Les sites XhoI et BamHI du vecteur navette E. coli-levure YEp13 Δ HindIII ont été éliminés de la manière suivante. Le plasmide a été traité avec BamHI. L'extension 5' a été remplie avec l'ADN polymérase de T4 et l'ADN a été recircularisé avec l'ADN ligase de T4 et utilisé pour transformer le E. coli MM294. Un plasmide récupéré à partir d'un des transformants a été traité comme ci-dessus sauf que l'enzyme de restriction était XhoI. Le vecteur pRIT10788 a été obtenu.

## G. pRIT10774

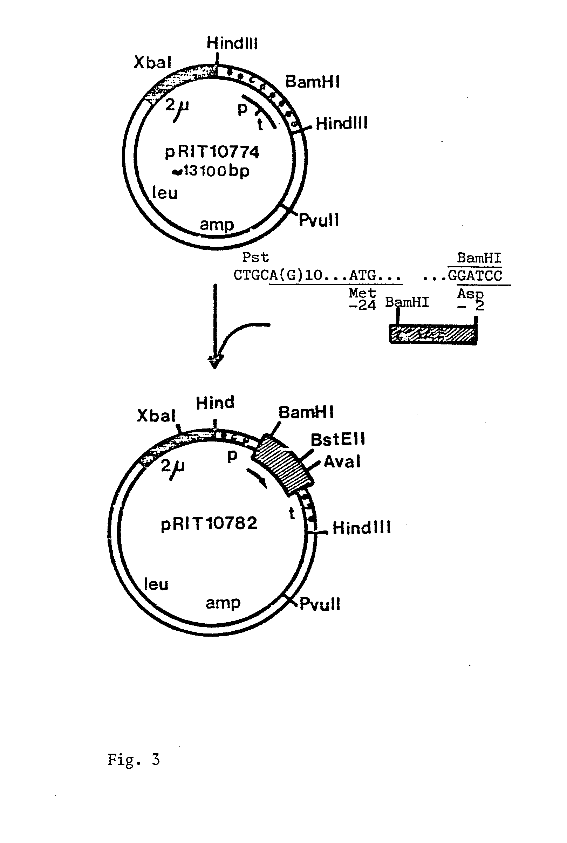

Le fragment HindIII de 2750 pb en provenance du pRIT10773 et contenant les éléments de régulation de l'Arg3 a été purifié par électrophorèse sur 1 % d'agarose suivie d'une électroélution et ligué avec le pRIT10788 qui avait été traité par HindIII et la phosphatase alcaline pour éviter la recircularisation. Après transformation du E. coli MM294, le vecteur d'expression pRIT10774 a été obtenu (Fig. 3).

Ce vecteur peut se répliquer à la fois dans E. coli et dans la levure et présente un site BamHI unique dans lequel des séquences codantes étrangères contenant un codon d'initiation de la traduction peuvent être insérées. Par suite des manipulations susmentionnées, ce vecteur comprend un fragment HindIII-XbaI du pYeUra3Arg3 portant l'Arg3tt.

Exemple 2

Pré-AATh dans pRIT10774

(Voir fig. 3) Le plasmide pULB1523 contient l'ADNc de l'AATh comprenant 14 pb de séquences non codantes en 5' et environ 100 pb de séquences non codantes en 3' clonées dans le site PstI du plasmide pBR322 par des extensions oligo dc/oligo dG homopolymériques. Ce plasmide est décrit dans le brevet belge n° 895961. L'insert du pULB1523 de 1400 pb a été purifié, après excision avec PstI, par électrophorèse sur un gel d'acrylamide à 7 % suivie d'une électroélution. Dix µg de fragment ont été traités par l'exonucléase Bal31 afin d'éliminer les nucléotides superflus en amont du codon de départ ATG de la pré-AATh. Après traitement par l'ADN polymérase de T4, 150 ng de la préparation résultante ont été mélangés avec du plasmide pRIT10774 (450 ng) qui avait été traité par BamHI et l'ADN polymérase de T4. Le mélange a été incubé pendant 16 h à 16° C avec l'ADN ligase de T4 et utilisé pour transformer la souche de E. coli et la rendre résistante à l'ampicilline.

Les plasmides de plusieurs transformants ont été analysés pour l'orientation de la séquence d'ADN de l'AATh. Les plasmides pRIT10782, 10783, 10789, 10790 et 10791, dans lesquels l'insert se trouve dans l'orientation correcte pour l'expression, ont été sélectionnés et transformés dans la souche lc1698d de <u>Saccharomyces</u> <u>cerevisiae</u> et les colonies leucine indépendante ont été sélectionnées. La souche lc1679d (<u>arg</u>J, <u>leu</u>2-1) est une souche bradytrophe pour l'arginine. Elle a été déposée à l'American Type Culture Collection, Rockville, Maryland, U.S.A. sous le numéro d'ordre 20631. La séquence à la jonction Arg3-AATh dans le pRIT10782 a été déterminée par le procédé de Maxam-Gilbert comme étant, en substance, comme suit :

5'          CCCATCAACTTGTACACTCG

TCTAGGATCGG ACAGTG AA

TCGACA<u>ATG</u>     CCG     TCT          3'

Par "en substance", on veut dire que la présente séquence peut différer par une ou quelques bases et qu'une ou quelques bases peuvent être substituées par suite d'un événement naturel ou dirigé sans modifier sensiblement l'activité de la molécule.

<p style="text-align:center">Exemple 3</p>

<p style="text-align:center">Expression de pRIT10782</p>

## A. Extraits

Les souches ont été cultivées sur un milieu de base azotée pour levure additionné de 4 $\mu$g/ml d'arginine jusqu'à ce que la densité optique atteigne 0,5-0,6 à 620 nm. Les cellules en provenance d'une culture de 350 ml ont été récupérées par centrifugation à 5000 G pendant 5 minutes et remises en suspension dans 5 ml de 100 mM de trométhamine-HCl (pH 7,4), 100 mM EDTA, 2 mM de fluorure de phényl méthyl sulphonyle (FPMS), 0,3 % de Triton X100. Les cellules ont été brisées par deux passages à travers une cellule de pression de French à 12.000 psi (81,7 MPa) et elles ont été brisées dans une presse de French. Les extraits ont ensuite été centri-

- 14 -                    0151102

fugés à 10.000 tpm pendant 40 minutes et les surnageants ont été analysés pour leur concentration en protéines en utilisant la méthode de Folin (les concentrations ont été ajustées à 3 mg/ml).

B. Détection immunologique de l'AATh dans les extraits

Les extraits de levure ont été analysés selon la méthode ELISA sandwich développée comme suit : Cent $\mu$l d'une solution de 8 $\mu$g/ml d'une fraction IgG en provenance d'anti-AATh (Immuno) de chèvre et purifiée dans le diéthylaminoéthylène (DEAE), dans une solution salée tamponnée au phosphate (PBS) à pH 8, ont été incubés pendant la nuit à 4° C avec, par réservoir, 100 $\mu$l soit d'un étalon AATh soit d'extraits de levure dilués dans une solution salée tamponnée au phosphate, pH 8, du sérum-albumine bovin, 1 % et du sérum de cheval, 10 % (PBS-BA-HS). Après plusieurs lavages au PBS (pH 8) contenant 0,1 % de Tween 80, 100 $\mu$l de sérum anti-AATh de souris à dilution 1/3000 dans du PBS-BAS-HS ont été ajoutés et incubés pendant la nuit à 4° C. Après plusieurs lavages, les anticorps liés de souris ont été révélés par les immunoglobulines de chèvre dressées contre les immunoglobulines de souris et marquées à la peroxydase (1 $\mu$g/ml dans PBS-BA-HS; 0,1 % de Tween 80) pendant 4 heures à température ordinaire et par le substrat chromogénique de l'enzyme (0,4 mg d'orthophénylènediamine et 0,2 mg/ml de peroxyde d'urée dans 0,1 M de tampon phosphate, pH 5), en substance, tel que décrit par O'Sullivan et al. Methods in Enzymology 73:147 (1981). L'absorption a été lue à 495 nm dans un lecteur automatique microElisa du type Dynatech AM120 (Herion et al., Bioscience Reports 3:381-388 (1983)). Une souche de levure portant le pRIT10782 s'est révélée produire la plus grande quantité de produit analogue à l'antitrypsine. Voir Tableau 1 qui suit.

Tableau 1.  Taux d'expression d'AATh dans la souche
1c1697d portant différents plasmides

| Plasmide | ng/ml extrait* | % de protéines totales |
|---|---|---|
| pRIT10774 | 1 | 0 |
| pRIT10789 | 6 | - |
| pRIT10790 | 6 | - |
| pRIT10782 | 24000 | 0,8 |
| pRIT10783 | 60 | - |
| pRIT10791 | 6 | - |

* ajusté à 3 mg/ml de protéines totales

Exemple 4

Caractérisation de l'AATh exprimée dans la souche :
1c1697d (pRIT10782) et 1c1697d (pRIT10783)

A. Extraits

Les culots de levure en provenance des cultures de 10 ml ont été lysés dans le dodécyl sulfate de sodium (DSS) comme suit. Les culots ont été remis en suspension dans un échantillon tampon contenant du DSS (50 mM de trométhamine-HCl, pH 7,6; DSS, 1 %, beta-mercapto-éthanol, 1 % et glycérol, 10 %), 0,3 % de Triton X100 et chauffés à ébullition pendant 3 minutes à 100° C. Les extraits ont été ensuite centrifugés pendant 10 minutes à 10.000 tpm. Ces échantillons peuvent être utilisés immédiatement pour l'analyse sur gel. Dans certains cas, les extraits ont été traités par 2 volumes d'acide acétique glacial et une concentration finale en acétate de magnésium 66 mM pendant 1 heure à 4° C. Après centrifugation pendant 30 minutes à 10.000 tpm, le surnageant a été traité par 10 volumes d'acétone (- 20° C) afin de précipiter les protéines. Celles-ci ont été centrifugées à 10.000 tpm; le culot a été séché et remis en suspension dans un tampon adapté à l'électrophorèse sur gel de DSS.

## B. Electrotransfert

L'électrophorèse sur gel de polyacrylamide a été effectuée en présence de DSS, en substance, ainsi qu'il a été décrit par Laemmli, Nature 227:680-685 (1970). Une plaque de gel de polyacrylamide dans du DSS en provenance de A ci-dessus a été lavée, pendant un court instant, à deux reprises avec 40 ml d'eau distillée et trois fois pendant 20 minutes chacune dans 50 mM de tampon phosphate, pH 7,5 (dégazé). Le transfert a été effectué sur des feuilles de nitrocellulose pendant deux heures à 10 V et 0,8 amp. dans le même tampon.

## C. Immunodétection sur filtres de transfert en nitrocellulose

La feuille de nitrocellulose en provenance de B, ci-dessus, a été saturée avec le tampon PBS, pH 7,5, contenant 0,1 % de Tween 80, 10 % de sérum de cheval et 1 % de sérum-albumine de boeuf et abandonnée pendant 4 heures à température ordinaire. Ensuite, la feuille a été lavée trois fois pendant 20 minutes chacune avec le tampon PBS, pH 7,5, avec 0,1 % de Tween 80 et incubée pendant la nuit à température ordinaire dans 25 ml du tampon de saturation additionné de sérum anti-AATh de souris (dilution 1/1000).

La feuille a ensuite été lavée 5 fois avec 40 ml de tampon PBS, pH 7,5, avec 0,1 % de Tween 80 pendant environ 2 heures et ensuite incubée pendant 4 heures à température ordinaire dans 25 ml de tampon PBS-Tween contenant 10 $\mu$g/ml de sérum de chèvre anti-souris marqué à la peroxydase (CAS-POD). La feuille a ensuite été lavée pendant deux heures dans le tampon PBS-Tween (5 fois, 40 ml). L'immunodétection a été réalisée en traitant la feuille de nitrocellulose pendant 25 minutes dans le substrat chromogénique (10 mg de diamino-benzidine et 100 $\mu$l de peroxyde d'urée (10 %) dans 50 ml de tampon trométhamine 0,1 M, pH 7,6). La feuille a finalement été lavée à l'eau distillée. Trois produits de 55, 53 et 44 kilodaltons ont été immunodétectés dans les extraits provenant de la souche pRIT10782. Ces dimensions ont été déterminées à partir d'une courbe de calibration basée sur la migration d'un poids moléculaire étalon. Les étalons ont été placés sur le même gel que l'extrait de levure.

### Exemple 5

### Reconnaissance de l'anticorps de l'AATh produite par la souche lcl697d (pRIT10782)

Les extraits de levure de lcl697d (pRIT10782) contenant une quantité définie de AATh ont été comparés à la même quantité de AATh naturelle selon la méthode ELISA sandwich. Le procédé était tel que décrit ci-dessus excepté que l'IgG de l'anti-AATh de chèvre a été remplacée par les anticorps monoclonaux anti-AAH dressés contre l'AATh provenant du plasma. Voir Herion et al., brevet belge n° 896543. Les résultats sont indiqués au tableau 2.

Tableau 2. Reconnaissance de l'AATh produite par la
souche 1c1697d (pRIT10782) par les anticorps
monoclonaux

| Anticorps monoclonaux | Rapport de liaison (%) : (AATh produit par la levure + AATh en provenance du plasma humain |
|---|---|
| AATY7, AATY8, AATY12, AATY13 AATY14, AATY15, AATY16, AATY17, AATY18, AATY19, AATY20, AATY21, AATY22, AATY23, AATY39, AATY42 | $> 85$ |
| AATY1, AATY6, AATY10 AATY11, AATY37, AATY38 | 40-65 |
| AATY3, AATY9 | 20-30 |
| AATY4, AATY5, AATY40 | 0-5 |

Exemple 6

Plasmide intermédiaire pRIT10779

Le plasmide pRIT10779 est un vecteur navette E. coli-levure portant une région promoteur et une région tt en provenance du gène de l'Arg3 séparée par un site BamHI. Le plasmide porte un codon d'initiation de la translation (ATG). Il a été préparé comme suit et est illustré dans la fig. 4.

Le plasmide pMC200 a été digéré par HincII et le fragment de 1940 pb contenant le promoteur Arg3 a été purifié par électrophorèse sur un gel d'acrylamide à 7 % suivie d'électroélution. Six µg de fragment ont été traités par l'exonucléase Bal31 afin d'éliminer les 2 nucléotides en aval de l'ATG de l'Arg3. Après traitement par l'ADN polymérase de T4 pour réparer toute extension à simple brin et digestion par XhoI, le mélange résultant de fragments (environ 750 pb de longueur, présentant d'un côté une extrémité fixe XhoI et différentes extrémités à bouts francs de l'autre côté, selon l'étendue de la digestion par Bal31) a été purifiée par électrophorèse sur un gel d'acrylamide à 7 % et électroélution.

Parallèlement, le plasmide pRIT10774 a été traité successivement par BamHI, par l'ADN polymérase de T4 (extensions BamHI à bouts francs) et par XhoI. Le fragment résultant de 12350 pb a été purifié par électrophorèse sur un gel d'agarose à 0,8 % suivie d'électroélution. Deux cents ng de ce fragment ont été mélangés avec 100 ng des fragments traités par Bal31, ligués par l'ADN ligase de T4 et le mélange a été utilisé pour transformer la souche de E. coli MM294 et la rendre résistante à l'ampicilline.

Les plasmides de plusieurs transformants ont été analysés pour la présence d'un site BamHI en position correcte, c'est-à-dire, adjacent au codon d'initiation de l'Arg3. Par suite de ces manipulations, la région promoteur est la même que dans les plasmides dérivés du pRIT10749 excepté que deux, plutôt qu'environ 13, nucléotides ont été supprimés en amont du site HincII dans le pYeUra3Arg3. L'Arg3 tt se trouve sur un fragment HaeIII-HindIII du pYeUra3Arg3 portant l'Arg3 tt. La séquence du plasmide choisi pRIT 10779 au site BamHI se présente, en substance, comme suit :

        5'      CCCATCAACTTGTACACTC

                GTTACCATG   GAT   CCC   CAG   3'

Ce plasmide peut se répliquer à la fois dans E. coli et dans la levure et il présente 2 sites uniques de restriction (BamHI et NcoI) où une séquence codante étrangère dépourvue d'un codon d'initiation peut y être insérée après traitement par BamHI et une nucléase, telle que la nucléase de la fève Mung ou après traitement par NcoI et une polymérase, telle que l'ADN polymérase de T4.

## Exemple 7

### AATh dans pRIT10779

La séquence codant pour l'AATh contient un site
BamHI recouvrant le codon pour le second acide aminé de
la protéine mature (Leicht et al. Nature 297:655-659
(1982). La phase de lecture en travers de ce site est
la même que dans le plasmide pRIT10779. En recombinant
ces sites BamHI, la phase de lecture a été conservée,
en permettant l'expression dans la levure d'une protéine identique à l'AATh excepté que le premier acide
aminé est la méthionine au lieu de l'acide glutamique.
Le site BamHI est unique dans le plasmide pRIT10782, en
permettant la construction suivante.

Le pRIT10782 a été digéré par XhoI et BamHI et le
plus grand fragment (13700 pb) a été purifié à partir
de gel d'agarose à 1 %. Parallèlement, le pRIT10779 a
été digéré par les mêmes enzymes et le plus petit fragment (750 pb) contenant le promoteur de l'Arg3 a été
purifié à partir d'un gel d'acrylamide. Trois cents ng
du fragment de 13700 pb ont été mélangés avec 100 ng du
fragment du 750 pb. Le mélange a été traité par l'ADN
ligase de T4 et utilisé pour transformer la souche
MM294 et la rendre résistante à l'ampicilline. Un
plasmide recombinant, le pRIT10787 présentant la structure correcte pour l'expression de l'ATTh a été obtenu
à partir d'un des transformants. La séquence à la
jonction Arg3-AATh a été déterminée selon le procédé de
Maxam-Gilbert
comme étant, en substance, comme suit :        5'
CCCATCAACTTGTACACTCGTTACCATG  GAT  CCC  CAG        3'

Exemple 8

Expression du pRIT10787

La souche lc1697d de levure a été transformée à l'aide du pRIT10787, en substance, ainsi qu'il a été décrit ci-dessus, et s'est révélée exprimer l'AATH mature ayant un méthionine N-terminal.

Exemple 9

Activité biologique de l'AATh produite dans les levures

La capacité inhibitrice de l'AATh humaine à l'égard de la trypsine dans un microdosage a été prise comme une mesure de son activité dans les extraits de levure. Le microdosage impliquait le clivage par la trypsine d'un substrat chromogénique en présence d'une préparation d'AATh étalon ou d'extraits dérivés des clones pRIT10774, pRIT10782 et pRIT10787. Le dosage a été effectué comme suit : des plaques de microdosage à 96 puits en polystyrène ont d'abord été incubées pendant 1 heure, avec une solution de sérum albumine bovin à 1 % et lavées ensuite de nombreuses fois avec de l'eau distillée. L'AATh a été incubée, à des concentrations variées, pendant 20 minutes à 37° C avec une quantité fixe de trypsine dans un volume réactionnel final de 200 µl (le tampon réactionnel se compose de 0,1 M de trométhamine HCl, pH 8,2, 0,15 M de NaCl, 0,01 M de EDTA et de polyéthylène glycol 6000 à 0,5 %). Les échantillons ont été refroidis lentement à température ambiante et exposés ensuite à un substrat chromogénique (20 µl d'une solution aqueuse 0,003 M de S2444 (chlorhydrate de L-pyroglutamyl-glycyl-L-argi-nine, p-nitroanilide Kabi Diagnostica - Suède). Au bout de 5 minutes à température ambiante, la réaction a été stoppée avec 50 ml d'acide acétique à 50 %. L'absorption a été relevée à 400 nm dans un lecteur automatique micro-ELISA (Dynatech AM120). Les courbes

- 22 -                                    0151102

de calibration ont été déterminées à la fois pour la trypsine et l'AATh. Les extraits de levure à tester ont été dilués en série dans un tampon réactionnel.

Les résultats sont fournis dans le tableau 3 qui suit.

| Tableau 3. Expression et activité de l'AATh dans les levures | | | | |
|---|---|---|---|---|
| Clone | Séquence codante | Souche | Taux d'expression (% de protéine totale) | Activité |
| pRIT10774 | aucune | 1c1697d | 0 | - |
| | | 10S44c | 0 | |
| pRIT10782 | pré-protéine | 1c1697d | 0,34 | - |
| | | 10S44c | 0,3 | - |
| pRIT10787 | protéine mature | 1c1697d | 1,2 | + |
| | | 10S44c | 1,0 | +++ |

(-) : aucune activité; (+) : activité modérée (au moins 10 % des molécules révèlent une activité; (+++) : activité plus élevée (au moins 30 % des molécules sont biologiquement actives).

Il faut noter que les données sur l'activité n'ont qu'une valeur qualitative puisque l'analyse est effectuée sur les extraits bruts de levure, lesquels pourraient contenir des inhibiteurs de l'AATh.

Aucune activité significative n'a été détectée dans les cellules produisant la pré-AATh. Cependant, l'AATh mature produite dans le clone pRIT10787 s'est révélée être active avec un optimum d'activité dans la souche de levure dépourvue de peptidase. Par comparaison avec la courbe d'activité étalon, l'AATh mature produite par la levure semble être au moins active à raison de 30 %. La pré-AATh peut être clivée par des techniques connues pour préparer l'AATh mature.

Exemple 10

### Caractérisation moléculaire de l'AATh produite dans la levure

Afin de confirmer l'identité des molécules d' AATh produites dans les levures, l'électrophorèse sur gel de polyacrylamide contenant DSS a été effectuée sur des extraits de levure provenant des clones pRIT10774, pRIT10782 et pRIT10787. Les protéines séparées ont été ensuite transférées électriquement sur des feuilles de nitrocellulose lesquelles, à leur tour, ont été traitées par du sérum anti-AATh de souris, du CAS-POD et un substrat chromogénique approprié. Le plasmide pRIT10787 dirige la synthèse d'un polypeptide simple ayant un poids moléculaire de 44.000 daltons, conforme au poids moléculaire de l'AATh mature non glycosylée. Le plasmide pRIT10782 qui code pour la préprotéine, dirige la synthèse de plusieurs polypeptides dans la levure : 1) une espèce mineure de 44.000 daltons qui pourrait être transformée en AATh; ii) une molécule de 47.000 daltons qui correspond à la préprotéine (protéine mature plus son peptide signal de 24 acides aminés) et iii) deux espèces plus lourdes de 50.000 et 53.000 daltons qui pourraient correspondre à l'AATh partiellement glycosylée.

Bien que les modes de réalisation préférés conformes à la présente invention soient totalement décrits ci-dessus, il va sans dire que la présente invention ne se limite pas à ces constructions décrites mais qu'elle comprend plutôt toutes les modifications entrant dans le plan des revendications suivantes.

Revendications pour les pays contractants :

BE, CH, DE, FR, GB, IT, LI, LU, NL, SE

1. Molécule d'ADN recombinante comprenant une séquence nucléotidique qui code pour l'alpha-1-anti-trypsine humaine (AATh) et une région de régulation qui peut effectuer la transcription de la séquence AATh dans la levure.

2. Molécule suivant la revendication 1, dans laquelle la région de régulation comprend le promoteur Arg3 de la levure.

3. Molécule suivant la revendication 1 ou 2, dans laquelle la séquence nucléotidique de l'AATh code pour l'AATh mature.

4. Molécule suivant la revendication 2, dans laquelle la région du promoteur ne comprend pas l'ATG de l'Arg3 et dans laquelle la région de régulation comprend en outre le terminateur de la transcription de l'Arg3.

5. Molécule suivant la revendication 4, dans laquelle la séquence AATh est un fragment PstI de 1370 pb portant 24 codons pour la région du précurseur de la pré-AATh.

6. Molécule suivant la revendication 5, dans laquelle la jonction séquentielle promoteur-AATh est, en substance, comme suit :

```
5'    CCCATCAACTTGTACACTCG
      TCTAGGATCGG ACAGTG AA
      TCGACAAATG  CCG   TCT    3'
```

7. Molécule suivant la revendication 5 qui porte les régions de régulation de l'Arg3 et la séquence codant pour l'AATh, en substance, telle que contenue dans le pRIT10782.

8. Molécule suivant la revendication 2, dans laquelle la région du promoteur comprend l'ATG de l'Arg3 et dans laquelle la région de régulation comprend en outre le terminateur de la transcription de l'Arg3.

9. Molécule suivant la revendication 8, dans laquelle la séquence AATh est un fragment BamHI-PstI qui débute à l'extrémité 5' avec le second acide aminé de l'AATh mature et qui s'insère immédiatement en aval de l'ATG de l'Arg3.

10. Molécule suivant la revendication 8, dans laquelle la jonction séquentielle promoteur-AATh est, en substance, comme suit :

    5'   CCCATCAACTTGTACACTC

         GTTACCATG   GAT   CCC   CAG   3'

11. Molécule suivant la revendication 9 qui porte la fusion Arg3-séquence codant pour l'AATh, en substance, telle que contenue dans le pRIT10787.

12. Microorganisme transformé par la molécule de l'une quelconque des revendications 1 à 11.

13. Vecteur d'expression plasmidique pour levure comprenant les régions du promoteur et du terminateur de la transcription de l'Arg3 et ayant un site de restriction endonucléasique entre les régions du promoteur et du terminateur pouor l'insertion et l'expression d'une séquence codante.

14. Vecteur suivant la revendication 13, qui porte les régions de l'Arg3, en substance, telles que contenues dans le pRIT10773.

15. Vecteur suivant la revendication 13, qui porte les régions de l'Arg3, en substance, telles que contenues dans le pRIT10774.

16. Vecteur suivant la revendication 13, qui porte les régions de l'Arg3, en substance, telles que contenues dans le pRIT10779.

17. Procédé de préparation d'une molécule d'ADN recombinante caractérisé en ce qu'on ligue une séquence nucléotidique qui code pour l'alpha-1-antitrypsine humaine (AATh) à une région de régulation qui peut effectuer la transcription de la séquence AATh dans la levure.

18. Procédé suivant la revendication 17, dans laquelle la région de régulation comprend le promoteur Arg3 de la levure.

19. Procédé suivant la revendication 17 ou 18, dans laquelle la séquence nucléotidique de l'AATh code pour l'AATh mature.

20. Procédé suivant la revendication 18, dans laquelle la région du promoteur ne comprend pas l'ATG de l'Arg3 et dans laquelle la région de régulation comprend en outre le terminateur de la transcription de l'Arg3.

21. Procédé suivant la revendication 20, dans laquelle la séquence AATh est un fragment PstI de 1370 pb portant 24 codons pour la région du précurseur de la pré-AATh.

22. Procédé suivant la revendication 21, dans laquelle la jonction séquentielle promoteur-AATh est, en substance, comme suit :

5'    CCCATCAACTTGTACACTCG

TCTAGGATCGG ACAGTG AA

TCGACAAATG   CCG   TCT      3'

23. Procédé suivant la revendication 21 dans laquelle la molécule porte les régions de régulation de l'Arg3 et la séquence codant pour l'AATh, en substance, est telle que contenue dans le pRIT10782.

24. Procédé suivant la revendication 18, dans laquelle la région du promoteur comprend l'ATG de l'Arg3 et dans laquelle la région de régulation comprend en outre le terminateur de la transcription de l'Arg3.

25. Procédé suivant la revendication 24, dans laquelle la séquence AATh est un fragment BamHI-PstI qui débute à l'extrémité 5' avec le second acide aminé de l'AATh mature et qui s'insère immédiatement en aval de l'ATG de l'Arg3.

26. Procédé suivant la revendication 24, dans laquelle la jonction séquentielle promoteur-AATh est, en substance, comme suit :

```
5'    CCCATCAACTTGTACACTC
      GTTACCATG   GAT   CCC   CAG   3'
```

27. Procédé suivant la revendication 25 dans laquelle la molécule porte la fusion Arg3-séquence codant pour l'AATh, en substance, telle que contenue dans le pRIT10787.

28. Procédé de transformation d'un microorganisme caractérisé en ce qu'on introduit dans des cellules de levure une molécule suivant l'une quelconque des revendications 1 à 11.

29. Procédé de préparation d'un vecteur d'expression plasmidique pour levure comprenant les régions du promoteur et du terminateur de la transcription de l'Arg3 caractérisé en ce qu'on introduit un site de restriction endonucléasique entre les régions du promoteur et du terminateur pour l'insertion et l'expression d'une séquence codante.

30. Procédé suivant la revendication 29, dans laquelle le vecteur porte les régions de l'Arg3, en substance, telles que contenues dans le pRIT10773.

31. Procédé suivant la revendication 29, dans laquelle le vecteur porte les régions de l'Arg3, en substance, telles que contenues dans le pRIT10774.

32. Procédé suivant la revendication 29, dans laquelle le vecteur porte les régions de l'Arg3, en substance, telles que contenues dans le pRIT10779.

Revendications pour le pays contractant AT

1. Procédé de préparation d'une molécule d'ADN recombinante caractérisé en ce qu'on ligue une séquence nucléotidique qui code pour l'alpha-1-antitrypsine humaine (AATh) à une région de régulation qui peut effectuer la transcription de la séquence AATh dans la levure.

2. Procédé suivant la revendication 1, dans laquelle la région de régulation comprend le promoteur Arg3 de la levure.

3. Procédé suivant la revendication 1 ou 2, dans laquelle la séquence nucléotidique de l'AATh code pour l'AATh mature.

4. Procédé suivant la revendication 2, dans laquelle la région du promoteur ne comprend pas l'ATG de l'Arg3 et dans laquelle la région de régulation comprend en outre le terminateur de la transcription de l'Arg3.

5. Procédé suivant la revendication 4, dans laquelle la séquence AATh est un fragment PstI de 1370 pb portant 24 codons pour la région du précurseur de la pré-AATh.

6. Procédé suivant la revendication 5, dans laquelle la jonction séquentielle promoteur-AATh est, en substance, comme suit :

5' CCCATCAACTTGTACACTCG

TCTAGGATCGG ACAGTG AA

TCGACAA<u>ATG</u> CCG TCT 3'

7. Procédé suivant la revendication 5 dans laquelle la molécule porte les régions de régulation de l'Arg3 et la séquence codant pour l'AATh, en substance, est telle que contenue dans le pRIT10782.

8. Procédé suivant la revendication 2, dans laquelle la région du promoteur comprend l'ATG de l'Arg3 et dans laquelle la région de régulation comprend en outre le terminateur de la transcription de l'Arg3.

9. Procédé suivant la revendication 8, dans laquelle la séquence AATh est un fragment BamHI-PstI qui débute à l'extrémité 5' avec le second acide aminé de l'AATh mature et qui s'insère immédiatement en aval de l'ATG de l'Arg3.

10. Procédé suivant la revendication 8, dans laquelle la jonction séquentielle promoteur-AATh est, en substance, comme suit :

5'    CCCATCAACTTGTACACTC

GTTACCATG   GAT   CCC   CAG   3'

11. Procédé suivant la revendication 9 dans laquelle la molécule qui porte la fusion Arg3-séquence codant pour l'AATh est, en substance, telle que contenue dans le pRIT10787.

12. Procédé de transformation d'un microorganisme caractérisé en ce qu'on introduit dans des cellules de levure une molécule obtenue suivant l'une quelconque des revendications 1 à 11.

13. Procédé de préparation d'un vecteur d'expression plasmidique pour levure comprenant les régions du promoteur et du terminateur de la transcription de l'Arg3 caractérisé en ce qu'on introduit un site de restriction endonucléasique entre les régions du promoteur et du terminateur pouor l'insertion et l'expression d'une séquence codante.

14. Procédé suivant la revendication 13, dans laquelle le vecteur porte les régions de l'Arg3, en substance, telles que contenues dans le pRIT10773.

15. Procédé suivant la revendication 13, dans laquelle le vecteur porte les régions de l'Arg3, en substance, telles que contenues dans le pRIT10774.

0151102

16. Procédé suivant la revendication 13, dans laquelle le vecteur porte les régions de l'Arg3, en substance, telles que contenues dans le pRIT10779.

Fig. 1

Fig. 2

Fig. 3

0151102

Fig. 4